# EUROPEAN PATENT SPECIFICATION

(11) **EP 3 941 593 B1**
(45) Date of publication and mention of the grant of the patent: **12.10.2022**
(21) Application number: 20708516.8
(22) Date of filing: 12.03.2020
(51) Int. Cl.: A61Q 15/00, A61K 8/19, A61K 8/24, A61K 8/27, A61K 8/28, A61K 8/365

(54) **AN ANTIPERSPIRANT COMPOSITION COMPRISING REACTIVE SALTS**
SCHWEISSHEMMENDE ZUSAMMENSETZUNG MIT REAKTIVEN SALZEN
COMPOSITION ANTISUDORIFIQUE COMPRENANT DES SELS RÉACTIFS

(30) Priority: 18.03.2019 WO PCT/CN2019/078550; 30.04.2019 EP 19171731
(43) Date of publication of application: 26.01.2022
(73) Proprietor: Unilever IP Holdings B.V., 3013 AL Rotterdam (NL); UNILEVER GLOBAL IP LIMITED, Wirral Merseyside CH62 4ZD (GB)
(72) Inventor: LIU, Renjiang, Shanghai, 200335 (CN); LI, Xiaoke, Shanghai, 200335 (CN); LIU, Weining, Shanghai, 200335 (CN); ZHOU, Huanjun, Shanghai, 200335 (CN)
(74) Representative: Whaley, Christopher
(86) International application number: PCT/EP2020/056651
(87) International publication number: WO 2020/187686

(56) References cited:
- WO-A1-98/10736
- WO-A1-2013/013903
- WO-A1-2016/105430
- WO-A1-2017/117301
- WO-A1-2019/072831
- WO-A2-2013/013902
- FR-A1- 2 978 033
- FR-A1- 2 978 034
- US-A- 5 895 641
- US-A1- 2003 026 768
- US-B1- 6 221 340

## Description

### Field of the invention

The present invention is in the field of antiperspirant compositions.

### Background of the invention

The present invention relates to compositions that contain antiperspirant actives. These actives are added to compositions to reduce perspiration upon topical application of the compositions to the body, particularly to the underarm regions of the human body viz. the axilla, and sometimes even on the upper part of the body near the chest. Usually, the conventional antiperspirant actives are salts of metals having an astringent effect, such as the salts of aluminium and/or zirconium. Since antiperspirants are used regularly, and have been used for decades, there is an ever-increasing need to develop alternative antiperspirant actives which are equally efficacious and safe.

FR2978034 (L'Oreal, 2011) discloses a multiphasic anti-perspirant emulsion comprising two separate reagents in two different phases of the emulsion and producing an antiperspirant effect when the emulsion is applied to the skin through in-situ reaction of the reagents on the skin.

WO13013903 A1 (L'Oreal, 2011) discloses a process for treating perspiration using an anhydrous composition comprising two reagents that together produce an antiperspirant effect in situ on the skin. The composition is anhydrous to prevent any reaction between the reagents in the pack.

WO13013902 A2 (L'Oreal, 2011) discloses a method for reducing perspiration which comprises mixing two compositions (A and B) which are packaged separately, and the application of the two compositions to skin simultaneously or sequentially. The composition A comprises at least a polyvalent cation salt and the composition B comprises at least an anion salt, and together they can form an antiperspirant salt *in situ* on the skin. Therefore, the compositions A and B are separately packaged.

WO 2019/072831 A1 discloses a cosmetic process for treating human perspiration and possibly body odour resulting from perspiration, comprising the use of at least one cation Xn+ of valence n, at least one anion Ym- of valence m and at least one modulating agent.

WO 2017/117301 A1 discloses an external personal care composition useful as an antiperspirant and a deodorant to occlude pores and to reduce sweat in a person and having a pH of 4.4 or 4.9, comprising tin(II) fluoride or tin(II) chloride as a water-soluble non-phosphate salt and a potassium or sodium tripolyphosphate salt as water-soluble phosphate salt. When the resulting salt/complex contacts sweat, it forms a precipitate allowing to occlude the pores and reduce the sweat. No retarding agent is present.

### Summary of the invention

It is known that water-soluble phosphate salts and water-soluble non-phosphate salts can react with each other by double displacement reaction to form a precipitate of a water-insoluble phosphate salt and which could act as an antiperspirant active. However, a technical problem is to keep them stable in one single formulation whilst still ensuring that composition remains efficacious at the time of application or use.

The present inventors have surprisingly observed that if a retarding agent in the composition, the abovementioned objects could be achieved. The retarding agent binds with the water-soluble phosphate salt or the water-soluble non-phosphate salt to retard or delay the double displacement reaction. Once the composition is applied to skin, the two salts diffuse into the sweat ducts before the double displacement reaction begins due to the presence of the retarding agent. When the composition contacts with sweat, which is an aqueous saline medium having pH from 5 to 7, the composition experiences an increase in pH conditions, which could, for example, be marginally higher than the pH of the composition itself, but the present inventors have found that such a change is sufficient to trigger the double displacement reaction. At this stage, it is believed that the double displacement reaction takes place inside the sweat ducts because the bond between the salt and the retarding agent is believed to have been sufficiently weakened. The double displacement reaction product, which in some cases manifests itself as a precipitate, acts like a plug which block sweat ducts partially or fully in a non-permanent manner and is thereby capable of preventing, or at least reducing, the production of sweat. In this manner, due to the *in-situ* formation of the water-insoluble salt, the compositions in accordance with the invention behaves like a conventional antiperspirant composition which usually contains compounds of metals such as aluminium.

In accordance with a first aspect is disclosed an aqueous antiperspirant composition having pH 2 to 5 comprising:
(i) a water-soluble phosphate salt;
(ii) a water-soluble non-phosphate salt; and,
(iii) a retarding agent.

wherein said water-soluble phosphate salt is selected from monosodium phosphate, disodium phosphate, trisodium phosphate, monopotassium phosphate, dipotassium phosphate, tripotassium phosphate, monoammonium phosphate, diammonium phosphate, triammonium phosphate, lithium dihydrogen phosphate, dilithium hydrogen phosphate and lithium phosphate; wherein said water-soluble non-phosphate salt is selected from calcium chloride, calcium gluconate, calcium nitrate, calcium bromide, calcium formate, calcium lactate, calcium propanoate, calcium acetate, calcium dobesilate, magnesium chloride, magnesium nitrate, magnesium sulphate, magnesium citrate, magnesium gluconate, magnesium acetate, magnesium lactate, magnesium malate, ferric chloride, ferric nitrate, ferric sulphate, zinc chloride, zinc nitrate, zinc citrate, zinc sulphate, zinc gluconate, copper chloride, copper sulphate, copper nitrate, copper(II) acetate, copper(II) gluconate, iron(III) citrate, zirconium nitrate, zirconium tetrachloride, zirconium oxychloride, strontium chloride, strontium nitrate, barium chloride, barium nitrate, lanthanum chloride, lanthanum acetate, Tin(II) chloride, Tin (IV) chloride, and silver nitrate; wherein said retarding agent is a compound which comprises at least one functional group selected from -COOH, -COO⁻, -SO₃H or -SO₃⁻; wherein said composition is not an oral care composition;
wherein said water-soluble phosphate salt, water-soluble non-phosphate salt and retarding agent are present in one phase; wherein the solubility of said salt in water is assessed at 25°C and 101.325 kPa (atmospheric pressure).

In accordance with a second aspect is disclosed a method of reducing perspiration comprising a step of topical application of the composition of the first aspect.

In accordance with a third aspect is disclosed use of the composition of the first aspect for reduction of bodily perspiration.

All other aspects of the present invention will more readily become apparent upon considering the detailed description and examples which follow.

All amounts are by weight of the antiperspirant composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Detailed description of the invention

By "An antiperspirant Composition" as used herein, is meant to include a composition for topical application to the skin of mammals, especially humans. Such a composition is preferably of the leave-on type. By a leave-on composition is meant a composition that is applied to the desired skin surface and left on for one minute to 24 hours after which it may be wiped or rinsed off with water, usually during the regular course of personal washing. The composition may also be formulated into a product which is applied to a human body for improving the appearance, cleansing, odor control or general aesthetics. The composition of the present invention can be in the form of a liquid, lotion, cream, foam, scrub, gel or stick form and may be delivered through a roll-on device or using a propellant containing aerosol can. "Skin" as used herein is meant to include skin on any part of the body (e.g., neck, chest, back, arms, underarms, hands, legs, buttocks and scalp) especially the underarms. The composition of the present invention is not a dentifrice, and certainly not an oral care composition.

### Solubility

"Water-soluble" and "water-insoluble" for the purpose of the present invention means the solubility of a salt (e.g., like calcium salts) in water at 25°C and 101.325 kPa (atmospheric pressure).

For the purpose of the present invention, water-soluble salts have a solubility of at least 0.1 moles per litre and water-insoluble salts have a solubility of less than 0.001 moles per litre, solubilities being measured at 25°C and 101.325 kPa (atmospheric pressure).

### Water-soluble phosphate salt and water-soluble non-phosphate salt

Antiperspirant compositions in accordance with this invention comprise a water-soluble phosphate salt and a water-soluble non-phosphate salt wherein the water-soluble phosphate salt and water-soluble non-phosphate salt are capable of reacting with each other by double displacement reaction to form a precipitate of a water-insoluble phosphate salt.

Double displacement is a type of chemical reaction where two compounds react, and the positive ions (cation) and the negative ions (anion) of the two reactants switch places, forming two new compounds or products.

The water-soluble phosphate salt of the present invention is selected from monosodium phosphate, disodium phosphate, trisodium phosphate, monopotassium phosphate, dipotassium phosphate, tripotassium phosphate, monoammonium phosphate, diammonium phosphate, triammonium phosphate, lithium dihydrogen phosphate, dilithium hydrogen phosphate and lithium phosphate.

It is preferred that the water-soluble phosphate salt is a sodium salt or potassium salt.

The water-soluble non-phosphate salt of the present invention is selected from calcium chloride, calcium gluconate, calcium nitrate, calcium bromide, calcium formate, calcium lactate, calcium propanoate, calcium acetate, calcium dobesilate, magnesium chloride, magnesium nitrate, magnesium sulphate, magnesium citrate, magnesium gluconate, magnesium acetate, magnesium lactate, magnesium malate, ferric chloride, ferric nitrate, ferric sulphate, zinc chloride, zinc nitrate, zinc citrate, zinc sulphate, zinc gluconate, copper chloride, copper sulphate, copper nitrate, copper(II) acetate, copper(II) gluconate, iron(III) citrate, zirconium nitrate, zirconium tetrachloride, zirconium oxychloride, strontium chloride, strontium nitrate, barium chloride, barium nitrate, lanthanum chloride, lanthanum acetate, Tin(II) chloride, Tin (IV) chloride, and silver nitrate.

It is preferred that the water-soluble non-phosphate salt is a calcium salt or magnesium salt.

It is preferred that the antiperspirant composition comprises from 0.1 to 40 wt%, and more preferably from 1 to 30 wt%, and most preferably from 5 to 20 wt% of the water-soluble phosphate salt, based on total weight of the composition.

It is preferred that the antiperspirant composition comprises from 0.1 to 40 wt%, and more preferably from 1 to 30 wt%, and most preferably from 5 to 20 wt% of the water-soluble non-phosphate salt, based on total weight of the composition.

It is particularly preferred that the water-soluble phosphate salt is sodium phosphate or potassium phosphate wherein the water-soluble non-phosphate salt is calcium chloride or magnesium chloride.

### Retarding agent

Antiperspirant compositions in accordance with this invention comprise a retarding agent to complex with the water-soluble phosphate salt or water-soluble non-phosphate salt to retard/delay the double displacement reaction when the pH is from 2 to 5, and where the complex is broken with the pH increasing when the antiperspirant composition is applied to skin. Therefore, it is essential to have the composition formulated and delivered at a low pH in the range of 2 to 5. Without wishing to be bound by theory it is believed that under certain pH (from 2 to 5), the compositions of the invention are stable because of the presence of the retarding agent and when the pH is increased to certain level (diverse for different compositions of the invention) during application, the double displacement reaction happens, and the composition is destabilized.

The retarding agent of the present invention is a compound which comprises at least one functional group selected from -COOH, -COO⁻, -SO₃H and -SO₃⁻.

When the retarding agent comprises a -COOH group, it is preferred that the retarding agent is selected from an amino acid and a carboxylic acid.

It is preferred that the amino acid is selected from arginine, asparagine, aspartic acid, cysteine, glutamic acid, glutamine, glycine, histidine, isoleucine, leucine, lysine, methionine, phenylalanine, proline, serine, threonine, tryptophan, tyrosine, valine and alanine.

It is preferred that the carboxylic acid is selected from fatty acid, acetic acid, malonic acid, anthranilic acid, glycolic acid, lactic acid, malic acid, citric acid, tartaric acid, succinic acid, benzoic acid, phthalic acid, chloroacetic acid, propionic acid, sorbic acid, salicylic acid, trichloroacetic acid, formic acid, gallic acid, maleic acid, thioglycolic acid and trifluoroacetic acid.

When the retarding agent comprises a -SO₃H group, it is preferred that the retarding agent is a sulfonic acid.

It is preferred that the sulfonic acid is selected from benzenesulfonic acid, sulfamic acid, sulfanilic acid, methanesulfonic acid and p-toluenesulfonic acid.

When the retarding agent comprises -COO⁻ or -SO₃⁻, it is preferred that the retarding agent is selected from sodium acetate, sodium citrate, sodium sorbate, sodium sulfamate, sodium alkylbenzenesulfonate, potassium citrate, potassium acetate, potassium sulfamate, potassium sorbate, potassium oxalate, potassium alkylbenzenesulfonate, ammonium acetate, ammonium citrate, ammonium sulfamate, ammonium oxalate, ammonium alkylbenzenesulfonate.

Without wishing to be bound by theory the inventors believe that the retarding agent can complex with the water-soluble phosphate salt or water-soluble non-phosphate salt to retard/delay said double displacement reaction. Once inside the sweat glands, it is believed that pH of the surrounding medium is about 5 to 7 because at this stage the composition comes in contact with sweat. This contact induces the breakdown of the complex and the double displacement reaction between the water-soluble phosphate salt and water-soluble non-phosphate salt to thereby form a precipitation, which partially or fully, and in a non-permanent manner, clogs or blocks the sweat ducts to provide antiperspirant benefits.

It is preferred that the antiperspirant composition comprises from 0.1 to 40 wt%, and more preferably from 0.5 to 30 wt%, and most preferably from 1 to 20 wt% of the retarding agent, based on total weight of the composition.

### Other ingredients

Other components commonly included in conventional antiperspirant compositions may also be incorporated in the compositions of the present invention. Such components include skin care agents such as emollients, humectants and skin barrier promoters; skin appearance modifiers such as skin lightening agents and skin smoothing agents; anti-microbial agents, in particular organic anti-microbial agents, and preservatives.

The antiperspirant compositions of the invention are applied cosmetically and topically to the skin, broadly speaking, by one of two methods. Different consumers prefer one method or the other. In one method, sometimes called a contact method, the composition is wiped across the surface of the skin, depositing a fraction of the composition as it passes. In the second method, sometimes called the non-contact method, the composition is sprayed from a dispenser held proximate to the skin, often in an area of about 10 to 20 cm². The spray can be developed by mechanical means of generating pressure on the contents of a dispenser, such as a pump or a squeezable sidewall or by internally generated pressure arising from a fraction of a liquefied propellant volatilizing, the dispenser commonly being called an aerosol.

There are broadly speaking two classes of contact compositions, one of which is liquid and usually applied using a roll-on dispenser or possibly absorbed into or onto a wipe, and in the second of which the antiperspirant active is distributed within a carrier liquid that forms a continuous phase that has been gelled. In one variation, the carrier fluid comprises a solvent for the antiperspirant and in a second variation, the antiperspirant remains a particulate solid that is suspended in an oil, usually a blend of oils.

The composition of the invention is aqueous and comprises cosmetically acceptable carrier. The term aqueous means that the composition of the invention comprises water as the main carrier or that water forms a major part of the carrier. In such cases, other solvents and ingredients other than water may also be present.

The composition of the invention can be an emulsion. When it is an emulsion, the water-soluble phosphate salt and the water-soluble non-phosphate salt are present in the same phase of the emulsion.

It is preferred that the composition of the invention is in the form of a lotion, a cream, a spray, a firm solid, a soft solid or is an emulsion packaged in a roll-on applicator.

Further preferably, when said composition is a spray it comprises a propellant and the composition is in the form of an aerosol.

### Stick compositions

In one aspect, the antiperspirant compositions of the invention is a stick composition which is usually in the form of an emulsion. Antiperspirant emulsion sticks are solidified compositions characterized as having aqueous and oil phases. Among such antiperspirant emulsion sticks are compositions having a disperse aqueous phase in which is dissolved an antiperspirant active, in usual cases, aluminium, zirconium and/or mixed aluminium/zirconium salts, and a continuous oil phase comprising one or more gelling agents capable of structuring such phase.

Antiperspirant emulsion sticks can be formulated as clear (i.e., translucent or transparent) or opaque compositions. Translucent or transparent emulsion sticks go on clear and, depending upon their formulation, may remain clear for extended periods of time, reducing the consumer perceived negative of "white marks" associated with deposition of antiperspirant active. Many different materials have been proposed as gellants for a continuous oil phase, including waxes, small molecule gelling agents and polymers. They each have their advantages and of them, one of the most popular class of gellants is waxes, partly at least due to their ready availability and ease of processing, including in particular linear fatty alcohol wax gellants. A gelled antiperspirant composition is applied topically to skin by wiping it across and in contact with the skin, thereby depositing on the skin a thin film.

The nature of the film depends to a significant extent on the gellant that is employed. Although wax fatty alcohols have been employed as gellants for many years, and are effective for the purpose of gelling, the resultant product is rather ineffective at improving the visual appearance of skin, and in particular underarm skin, to which the composition has been applied. This problem has been solved by including ameliorating materials for example, di or polyhydric humectants and/or a triglyceride oil.

Stick compositions are usually available in the form of a firm solid or a soft solid. Firm solids, as the name indicates, are harder and can be directly applied by way of an applicator, for example, to the underarms. Soft solids also need an applicator which is similar to the firm solids, the difference being that the soft solids are softer and the applicator needs to be designed in order to permit extrusion of the solids through a cap member comprising plurality of orifices and the extruded composition can then be applied to the underarms.

### Roll-on

Alternatively, the composition of the invention is a liquid composition, that can be dispensed from a roll-on package. Broadly speaking such compositions could be divided into two classes, namely those in which an antiperspirant active is suspended in a hydrophobic carrier, such as a volatile silicone and those in which the antiperspirant active is dissolved in a carrier liquid.

The second category of formulations that is an alternative to alcoholic formulations comprise a dispersion of water-insoluble or very poorly water-soluble ingredients in an aqueous solution of the antiperspirant. Herein, such compositions will be called emulsions. Antiperspirant roll-on emulsions commonly comprise one or more emulsifiers to maintain a distribution of the water-soluble ingredients.

### Aerosol compositions

Further alternatively, the antiperspirant composition of the invention is delivered through an aerosol composition which comprises a propellant in addition to the applicable other ingredients described hereinabove. Commonly, the propellant is employed in a weight ratio to the base formulation of from 95:5 to 5:95. Depending on the propellant, in such aerosol compositions the ratio of propellant to base formulation is normally at least 20:80, generally at least 30:70, particularly at least 40:60, and in many formulations, the weight ratio is from 90:10 to 50:50. A ratio range of from 70:30 to 90:10 is sometimes preferred.

Propellants herein generally are one of three classes; (i) low boiling-point gasses liquified by compression, (ii) volatile ethers and (iii) compressed non-oxidising gases.

Class (i) is conveniently a low boiling-point material, typically boiling below -5°C, and often below -15°C, and in particular, alkanes and/or halogenated hydrocarbons. This class of propellant is usually liquefied at the pressure in the aerosol canister and evaporates to generate the pressure to expel the composition out of the canister. Examples of suitable alkanes include particularly propane, butane or isobutane. The class (ii) of propellant comprises a very volatile ether of which the most widely employed ether hitherto is dimethyl ether. This propellant can advantageously be employed at relatively low weight ratio of propellant to base formulation, for example to as low as 5:95. It can also be employed in admixture with, for example, compressible/liquefiable alkane gasses. The class (iii) of propellant comprises compressed non-oxidising gasses, and in particular carbon dioxide or nitrogen. Inert gases like neon are a theoretical alternative.

The composition of the present invention can comprise a wide range of other optional components. The CTFA Personal Care Ingredient Handbook, Second Edition, 1992, describes a wide variety of non-limiting personal care and pharmaceutical ingredients commonly used in the skin care industry, which are suitable for use in the compositions of the present invention. Examples include: antioxidants, binders, biological additives, buffering agents, colorants, thickeners, polymers, astringents, fragrance, conditioners, exfoliating agents, pH adjusters, other than the ones already discussed earlier, preservatives, natural extracts, essential oils, skin sensates, skin soothing agents, and skin healing agents.

A preservative is a preferred additional component in compositions of the invention. A preservative serves to reduce or eliminate microbial contamination of compositions of the invention. Preservatives are typically employed at a total level of from 0.05 to 3%, preferably at from 0.1 to 2% and most preferably at from 0.4 to 1%.

Suitable preservatives for use with the present invention include 2-phenoxyethanol, iodopropynyl butylcarbamate, C₁-C₃ alkyl parabens, sodium benzoate, caprylyl glycol and EDTA. Particularly preferred preservatives are 2-phenoxyethanol, iodopropynyl butylcarbamate, sodium benzoate, caprylyl glycol and EDTA and especially preferred are 2-phenoxyethanol and iodopropynyl butylcarbamate.

A preferred additional component of compositions of the invention is a fragrance.

Suitable materials include conventional perfumes, such as perfume oils and also include so-called deo-perfumes, as described in EP 545,556 and other publications. Levels of incorporation are preferably up to 4% by weight, particularly from 0.1% to 2% by weight, and especially from 0.7% to 1.7% by weight.

An antimicrobial deodorant active is a preferred an additional component in compositions of the invention. Such components serve to reduce or eliminate body odour by reducing or otherwise impeding the function of microbes on the skin of the body responsible for malodour generation.

The antimicrobial deodorant active may also be a preservative for the composition.

When employed, the anti-microbial deodorant agent is typically incorporated into the composition at from 0.01% to 3% and particularly at from 0.03% to 0.5%.

Preferred anti-microbial deodorant agents have a minimum inhibitory concentration (MIC) of 1 mg.ml⁻¹ or less, particularly 200 µg.ml⁻¹ or less, and especially 100 µg.ml⁻¹ or less. The MIC of an anti-microbial agent is the minimum concentration of the agent required to significantly inhibit microbial growth. Inhibition is considered "significant" if an 80% or greater reduction in the growth of an inoculum of *Staphylococcus epidermidis* is observed, relative to a control medium without an anti-microbial agent, over a period of 16 to 24 hours at 37°C. Details of suitable methods for determining MICs can be found in "Antimicrobial Agents and Susceptibility Testing", C.Thornsberry, (in "Manual of Clinical Microbiology", 5th Edition, Ed. A. Balows et al, American Society for Microbiology, Washington D.C., 1991). A particularly suitable method is the Macrobroth Dilution Method as described in Chapter 110 of above publication (pp. 1101-1111) by D. F. Sahm and J. A. Washington II. MICs of anti-microbials suitable for inclusion in the compositions of the invention are triclosan: 0.01-10 µg.ml⁻¹ (J.Regos et al., Dermatologica (1979), 158: 72-79) and farnesol: ca. 25 µg.ml⁻¹ (K. Sawano, T. Sato, and R. Hattori, Proceedings of the 17th IFSCC International Conference, Yokahama (1992) p.210-232). By contrast ethanol and similar alkanols have MICs of greater than 1 mg.ml⁻¹.

Suitable organic anti-microbials are bactericides, for example quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred anti-microbials for use in the compositions of the invention are polyhexamethylene biguanide salts (also known as polyaminopropyl biguanide salts), an example being Cosmocil CQ^{™} available from Zeneca PLC, preferably used at up to 1% and more preferably at 0.03% to 0.3% by weight; 2',4,4'-trichloro,2-hydroxy-diphenyl ether (triclosan), preferably used at up to 1% by weight of the composition and more preferably at 0.05-0.3%; and 3,7,11-trimethyldodeca-2,6,10-trienol (farnesol), preferably used at up to 1% by weight of the composition and more preferably at up to 0.5%. Other suitable organic antimicrobial agents are transition metal chelators, as described in WO01/52805, for example. Transitional metal chelators having a binding coefficient for iron(III) of greater than 10²⁶, for example diethylenetriaminepentaacetic acid and salts thereof are preferred.

### Method and Use

The present invention also provides for a method of reducing perspiration comprising a step of topical application of the composition of the first aspect. Such topical application excludes the application to the oral cavity. Preferably, the present invention provides for a method wherein the composition of the first aspect is applied on the underarms. The present invention also provides for a method wherein said water-insoluble phosphate salt partially or fully blocks sweat ducts in a non-permanent manner. The method in accordance with the invention is preferably non-therapeutic. By non-therapeutic is meant that the method is cosmetic in nature.

The invention also provides for use of the composition of the first aspect for reduction of bodily perspiration. The use in accordance with the invention is preferably non-therapeutic in nature, more preferably cosmetic in nature. Preferably, the present invention provides for use wherein upon said use the sweat ducts present in the skin get partially or fully blocked in a non-permanent manner.

The invention will now be demonstrated with the help of the following non-limiting examples.

### Examples

### Example 1: Precipitation formation of compositions triggered by pH.

The following compositions were prepared as given in Table -1.

**Table -1**

| Reference No. | Water-soluble phosphate salt | Water-soluble non-phosphate salt | Retarding agent | Water |
|---|---|---|---|---|
| 1 | 6.3 wt% NaH₂PO₄ | 5.8 wt% CaCI2 | 4.7 wt% DL-malic acid | To 100 wt% |
| 2 | 6.3 wt% NaH₂PO₄ | 5.8 wt% CaCI2 | 4.7 wt% Citric acid | To 100 wt% |
| 3 | 6.3 wt% NaH₂PO₄ | 10.6 wt% MgCl₂·6H₂O | 4.7 wt% Glycolic acid | To 100 wt% |

The pH of the compositions was adjusted to 2.0 using 20 wt% phosphoric acid

The ability of the above compositions to form a precipitation was tested by the following procedure:
10 ml of the concerned composition was taken in a beaker and an aqueous solution of sodium hydroxide (3.0mol/L) was added dropwise under stirring, to increase the pH of the medium. The appearance of each sample was checked visually and recorded at various pH as the pH increased gradually. The samples were graded as either Clear (C), Turbid (T) or Precipitate (P). Turbid hereby means cloudy, opaque, or thick with suspended matter, which is considered as the starting point for the precipitation. Therefore, the precipitation formation sets in as soon as the appearance of sample turns into turbid.

The data is summarized in Table -2 below:

**Table -2**

| Reference No. | pH=2 | pH = 3 (with NaOH) | pH=3.9 (with NaOH) | pH =4.8 (with NaOH) | pH = 5.6 (with NaOH) |
|---|---|---|---|---|---|
| 1 | C | C | C | T | T |
| 2 | C | C | C | T | T |
| 3 | C | C | C | C | T |

The data in Table -2 above indicates that compositions as per the invention (Reference No. 1 and 2) were stable (clear solution) at pH 2, 3, 3.9 and their precipitations were triggered by an increase in the pH of the medium (pH = 4.8). The data in Table -2 above also indicates that compositions as per the invention (Reference No. 3 and 4) were stable at pH 2, 3, 3.9, 4.8 and their precipitations were triggered by an increase in the pH of the medium (pH = 5.6). It indicates the ability of the composition as per the invention to form a gel upon contact with sweat. It indicates the ability of the composition as per the invention to form a precipitation upon contact with model sweat whilst being transparent at pH of 2 to 5, which is the pH at which such antiperspirant compositions are usually formulated.

### Example 2: Effect of retarding agent

The following compositions were prepared as given in Table -3.

**Table -3**

| Reference No. | Water-soluble phosphate salt | Water-soluble non-phosphate salt | Retarding agent | Water |
|---|---|---|---|---|
| A | 5.0 wt% KH₂PO4 | 2.5 wt%CaCl₂ | -- | To 100 wt% |
| 5 | 5.0 wt% KH₂PO₄ | 2.5 wt%CaCl₂ | 5.0 wt% DL-malic acid | To 100 wt% |

The pH of the compositions was adjusted to 2.0 using 20 wt% phosphoric acid

The precipitation formation behaviour of above compositions was tested by following the procedure described earlier after various time periods. It can be noticed that the Reference compositions A is devoid of any retarding agent (therefore they are outside the present invention) whereas the corresponding compositions 5 contains a retarding. Therefore, the data or observations recorded at the end of this experiment is useful to appreciate the role of the retarding agent in the compositions according to the invention. The samples were graded as either Clear(C), Turbid (T) or Precipitate (P). The data is summarized in Table -4 below:

**Table -4**

| Reference no. | Time | pH=3.0 (with NaOH) | pH = 3.5 (with NaOH) | pH=3.8 (with NaOH) | pH = 4.02 (with NaOH) |
|---|---|---|---|---|---|
| A | 1 minute | P | P | P | P |
| 5 | 1 minute | C | C | C | C |
| | After 1 day | C | C | C | P |
| | After 3 days | C | C | C | P |
| | After 1 week | C | C | T | P |
| | After 2 weeks | C | C | T | P |

The data in Table -4 above indicates that compositions as per the invention (Reference No. 5) are capable of being stable at pH= 3, 3.5 and forming a precipitation quite slowly with pH increasing, while compositions outside the invention (Reference No. A) do not exhibit this property. It indicates that the existence of retarding agent can delay the reaction between these two salts and enable the ions of these two salts to diffuse into the sweat duct before precipitations being formed by the double replacement reaction, ensuring the precipitations formed can block the sweat duct effectively.

The composition of model ionic sweat (pH 6.9) is as given below in Table -5:

**Table -5**

| Ingredient | wt% of total |
|---|---|
| Potassium Chloride | 0.0373 |
| Sodium Bicarbonate | 0.2025 |
| Sodium Chloride | 0.2098 |
| Ammonium Chloride | 0.0107 |
| Calcium Chloride | 0.0222 |
| Lactic Acid | 0.0901 |
| Urea | 0.0018 |
| Water | 99.4256 |

The precipitation formation behaviour of the compositions in Table -5 was tested by following the procedure:
10 ml of the concerned composition was taken in a beaker and an aqueous solution of sodium hydroxide (3.0mol/L) was added dropwise under stirring, to increase the pH of the medium to pH=3.5. Then 0.2mL above solution is taken and mixed with 4ml model ionic sweat. The appearance of each sample was checked visually and recorded at certain pH level (measured) after various time periods. The samples were graded as either Clear (C), Turbid (T) or Precipitate (P).

The following compositions were prepared as given in Table -6.

**Table -6**

| Reference no. | Time | pH=4.9 (with model ionic sweat) |
|---|---|---|
| A | 1 minute | P |
| 5 | 1 minute | C |
| | After 1 day | T |
| | After 3 days | T |

The data in Table -6 above indicates that compositions as per the invention (Reference No. 5) are capable of forming a precipitation quite slowly after contacting with sweat, while compositions outside the invention (Reference No. A) do not exhibit this property. It indicates that the existence of retarding agent can delay the reaction between these two salts and enable the ions of these two salts to diffuse into the sweat duct before precipitations being formed by the double replacement reaction, ensuring the precipitations formed can block the sweat duct effectively.

## Claims

1. An aqueous antiperspirant composition having pH 2 to 5, comprising:
(i) a water-soluble phosphate salt;
(ii) a water-soluble non-phosphate salt; and,
(iii) a retarding agent;
wherein said water-soluble phosphate salt is selected from monosodium phosphate, disodium phosphate, trisodium phosphate, monopotassium phosphate, dipotassium phosphate, tripotassium phosphate, monoammonium phosphate, diammonium phosphate, triammonium phosphate, lithium dihydrogen phosphate, dilithium hydrogen phosphate and lithium phosphate; wherein said water-soluble non-phosphate salt is selected from calcium chloride, calcium gluconate, calcium nitrate, calcium bromide, calcium formate, calcium lactate, calcium propanoate, calcium acetate, calcium dobesilate, magnesium chloride, magnesium nitrate, magnesium sulphate, magnesium citrate, magnesium gluconate, magnesium acetate, magnesium lactate, magnesium malate, ferric chloride, ferric nitrate, ferric sulphate, zinc chloride, zinc nitrate, zinc citrate, zinc sulphate, zinc gluconate, copper chloride, copper sulphate, copper nitrate, copper(II) acetate, copper(II) gluconate, iron(III) citrate, zirconium nitrate, zirconium tetrachloride, zirconium oxychloride, strontium chloride, strontium nitrate, barium chloride, barium nitrate, lanthanum chloride, lanthanum acetate, Tin(II) chloride, Tin (IV) chloride, and silver nitrate; wherein said retarding agent is a compound which comprises at least one functional group selected from -COOH, -COO⁻, -SO₃H or -SO₃⁻; wherein said composition is not an oral care composition; wherein said water-soluble phosphate salt, water-soluble non-phosphate salt and retarding agent are present in one phase; wherein the solubility of said salt in water is assessed at 25°C and 101.325 kPa (atmospheric pressure).

2. An aqueous antiperspirant composition as claimed in claim 1 wherein said water-soluble non-phosphate salt is a calcium or magnesium salt.

3. An aqueous antiperspirant composition as claimed in claim 1 or 2 wherein said retarding agent comprises -COOH group and is selected from an amino acid and a carboxylic acid.

4. An aqueous antiperspirant composition as claimed in claim 1 or 2 wherein said retarding agent comprises -SO₃H group, and is selected from benzenesulfonic acid, sulfamic acid, sulfanilic acid, methanesulfonic acid and p-toluenesulfonic acid.

5. An aqueous antiperspirant composition as claimed in claim 1 or 2 wherein said retarding agent comprises -COO⁻ or -SO₃⁻ group, and is selected from sodium acetate, sodium citrate, sodium sorbate, sodium sulfamate, sodium alkylbenzenesulfonate, potassium citrate, potassium acetate, potassium sulfamate, potassium sorbate, potassium oxalate, potassium alkylbenzenesulfonate, ammonium acetate, ammonium citrate, ammonium sulfamate, ammonium oxalate, ammonium alkylbenzenesulfonate.

6. An aqueous antiperspirant composition as claimed in any of claims 1 to 5 wherein said composition comprises 0.1 to 40 wt% of said water-soluble phosphate salt.

7. An aqueous antiperspirant composition as claimed in any of claims 1 to 6 wherein said composition comprises 0.1 to 40 wt% of said water-soluble non-phosphate salt.

8. An aqueous antiperspirant composition as claimed in any of claims 1 to 7 wherein said composition comprises 0.1 to 40 wt% of said retarding agent.

9. An aqueous antiperspirant composition as claimed in any of claims 1 to 8 wherein said composition comprises an antimicrobial deodorant active.

10. An aqueous antiperspirant composition as claimed in any of claims 1 to 9 wherein said composition is in the form of a lotion, a cream, a spray, a firm solid, a soft solid or is an emulsion packaged in a roll-on applicator.

11. A method of reducing perspiration comprising a step of topical application of a composition as claimed in any of claims 1 to 10.

12. A method as claimed in claim 11 wherein said composition is applied to the underarms.

13. A method as claimed in claim 11 or 12 wherein said water-insoluble phosphate salt blocks sweat ducts partially or fully in a non-permanent manner.

14. Use of composition as claimed in any of claims 1 to 10 for reduction of bodily perspiration.

15. Use as claimed in claim 14 wherein upon said use the sweat ducts present in the skin get partially or fully blocked in a non-permanent manner.

## Patentansprüche

1. Wässrige schweißhemmende Zusammensetzung mit einem pH-Wert von 2 bis 5, umfassend:
(i) ein wasserlösliches Phosphatsalz;
(ii) ein wasserlösliches Nicht-Phosphat-Salz; und
(iii) ein Verzögerungsmittel;
wobei das wasserlösliche Phosphatsalz ausgewählt ist aus Mononatriumphosphat, Dinatriumphosphat, Trinatriumphosphat, Monokaliumphosphat, Dikaliumphosphat, Trikaliumphosphat, Monoammoniumphosphat, Diammoniumphosphat, Triammoniumphosphat, Lithiumdihydrogenphosphat, Dilithiumhydrogenphosphat und Lithiumphosphat; wobei das wasserlösliche Nicht-Phosphat-Salz ausgewählt ist aus Calciumchlorid, Calciumgluconat, Calciumnitrat, Calciumbromid, Calciumformiat, Calciumlactat, Calciumpropanoat, Calciumacetat, Calciumdobesilat, Magnesiumchlorid, Magnesiumnitrat, Magnesiumsulfat, Magnesiumcitrat, Magnesiumgluconat, Magnesiumacetat, Magnesiumlactat, Magnesiummalat, Eisenchlorid, Eisen(III)nitrat, Eisen(III)sulfat, Zinkchlorid, Zinknitrat, Zinkcitrat, Zinksulfat, Zinkgluconat, Kupferchlorid, Kupfersulfat, Kupfernitrat, Kupfer(II)-acetat, Kupfer(II)-gluconat, Eisen(III)-citrat, Zirkoniumnitrat, Zirkoniumtetrachlorid, Zirkoniumoxychlorid, Strontiumchlorid, Strontiumnitrat, Bariumchlorid, Bariumnitrat, Lanthanchlorid, Lanthanacetat, Zinn(II)chlorid, Zinn(IV)chlorid und Silbernitrat; wobei das Verzögerungsmittel eine Verbindung ist, die mindestens eine funktionelle Gruppe, ausgewählt aus -COOH, -COO⁻, -SO₃H oder -SO₃⁻, umfasst, wobei die Zusammensetzung keine Mundpflegezusammensetzung ist; wobei das wasserlösliche Phosphatsalz, das wasserlösliche Nicht-Phosphatsalz und das Verzögerungsmittel in einer Phase vorhanden sind; wobei die Löslichkeit des Salzes in Wasser bei 25°C und 101.325 kPa (Atmosphärendruck) bestimmt wird.

2. Wässrige schweißhemmende Zusammensetzung nach Anspruch 1, wobei das wasserlösliche Nicht-Phosphatsalz ein Calcium- oder Magnesiumsalz ist.

3. Wässrige schweißhemmende Zusammensetzung nach Anspruch 1 oder 2, wobei das Verzögerungsmittel eine -COOH-Gruppe umfasst und ausgewählt ist aus einer Aminosäure und einer Carbonsäure.

4. Wässrige schweißhemmende Zusammensetzung nach Anspruch 1 oder 2, wobei das Verzögerungsmittel eine -SO₃H-Gruppe umfasst und ausgewählt ist aus Benzolsulfonsäure, Sulfaminsäure, Sulfanilsäure, Methansulfonsäure und p-Toluolsulfonsäure.

5. Wässrige schweißhemmende Zusammensetzung nach Anspruch 1 oder 2, wobei das Verzögerungsmittel eine -COO⁻- oder -SO₃⁻-Gruppe umfasst und ausgewählt ist aus Natriumacetat, Natriumcitrat, Natriumsorbat, Natriumsulfamat, Natriumalkylbenzolsulfonat, Kaliumcitrat, Kaliumacetat, Kaliumsulfamat, Kaliumsorbat, Kaliumoxalat, Kaliumalkylbenzensulfonat, Ammoniumacetat, Ammoniumcitrat, Ammoniumsulfamat, Ammoniumoxalat und Ammoniumalkylbenzensulfonat.

6. Wässrige schweißhemmende Zusammensetzung nach irgendeinem der Ansprüche 1 bis 5, wobei die Zusammensetzung von 0,1 bis 40 Gew.-% des wasserlöslichen Phosphatsalzes umfasst.

7. Wässrige schweißhemmende Zusammensetzung nach irgendeinem der Ansprüche 1 bis 6, wobei die Zusammensetzung von 0,1 bis 40 Gew.-% des wasserlöslichen Nicht-Phosphatsalzes umfasst.

8. Wässrige schweißhemmende Zusammensetzung nach irgendeinem der Ansprüche 1 bis 7, wobei die Zusammensetzung von 0,1 bis 40 Gew.-% des Verzögerungsmittels umfasst.

9. Wässrige schweißhemmende Zusammensetzung nach irgendeinem der Ansprüche 1 bis 8 wobei die Zusammensetzung einen antimikrobiellen desodorierenden Wirkstoff umfasst.

10. Wässrige schweißhemmende Zusammensetzung nach irgendeinem der Ansprüche 1 bis 9, wobei die Zusammensetzung in Form einer Lotion, einer Creme, eines Sprays, eines harten Feststoffs, eines weichen Feststoffs oder einer Emulsion, die in einem Roll-on-Applikator abgefüllt ist, vorliegt.

11. Verfahren zur Verringerung der Transpiration, umfassend einen Schritt der topischen Anwendung einer Zusammensetzung nach einem der Ansprüche 1 bis 10.

12. Verfahren nach Anspruch 11, wobei die Zusammensetzung auf die Achselhöhlen aufgetragen wird.

13. Verfahren nach Anspruch 11 oder 12, wobei das wasserunlösliche Phosphatsalz die Schweißkanäle teilweise oder vollständig auf nicht dauerhafte Weise blockiert.

14. Verwendung der Zusammensetzung nach irgendeinem der Ansprüche 1 bis 10 zur Verringerung der Körperschweißbildung.

15. Verwendung nach Anspruch 14, wobei bei dieser Verwendung die in der Haut vorhandenen Schweißkanäle teilweise oder vollständig auf nicht dauerhafte Weise blockiert werden.

## Revendications

1. Composition d'antiperspirant aqueuse ayant un pH de 2 à 5, comprenant :
(i) un sel de phosphate soluble dans l'eau ;
(ii) un sel de non-phosphate soluble dans l'eau ; et,
(iii) un agent retardateur ;
dans laquelle ledit sel de phosphate soluble dans l'eau est choisi parmi le phosphate de monosodium, phosphate de disodium, phosphate de trisodium, phosphate de monopotassium, phosphate de dipotassium, phosphate de tripotassium, phosphate de monoammonium, phosphate de diammonium, phosphate de triammonium, dihydrogénophosphate de lithium, hydrogénophosphate de dilithium et phosphate de lithium ; dans laquelle ledit sel de non-phosphate soluble dans l'eau est choisi parmi le chlorure de calcium, gluconate de calcium, nitrate de calcium, bromure de calcium, formate de calcium, lactate de calcium, propanoate de calcium, acétate de calcium, dobésilate de calcium, chlorure de magnésium, nitrate de magnésium, sulfate de magnésium, citrate de magnésium, gluconate de magnésium, acétate de magnésium, lactate de magnésium, malate de magnésium, chlorure ferrique, nitrate ferrique, sulfate ferrique, chlorure de zinc, nitrate de zinc, citrate de zinc, sulfate de zinc, gluconate de zinc, chlorure de cuivre, sulfate de cuivre, nitrate de cuivre, acétate de cuivre (II), gluconate de cuivre (II), citrate de fer (III), nitrate de zirconium, tétrachlorure de zirconium, oxychlorure de zirconium, chlorure de strontium, nitrate de strontium, chlorure de barium, nitrate de barium, chlorure de lanthane, acétate de lanthane, chlorure d'étain (II), chlorure d'étain (IV), et nitrate d'argent ; dans laquelle ledit agent retardateur est un composé qui comprend au moins un groupe fonctionnel choisi parmi -COOH, -COO⁻, -SO₃H ou -SO₃⁻ ; dans laquelle ladite composition n'est pas une composition pour le soin oral ; dans laquelle lesdits sel de phosphate soluble dans l'eau, sel de non-phosphate soluble dans l'eau et agent retardateur sont présents dans une phase ; dans laquelle la solubilité dudit sel dans l'eau est estimée à 25°C et 101,325 kPa (pression atmosphérique).

2. Composition d'antiperspirant aqueuse selon la revendication 1, dans laquelle ledit sel de non-phosphate soluble dans l'eau est un sel de calcium ou de magnésium.

3. Composition d'antiperspirant aqueuse selon la revendication 1 ou 2, dans laquelle ledit agent retardateur comprend un groupe -COOH et est choisi parmi un acide aminé et un acide carboxylique.

4. Composition d'antiperspirant aqueuse selon la revendication 1 ou 2, dans laquelle ledit agent retardateur comprend un groupe -SO₃H, et est choisi parmi l'acide benzènesulfonique, acide sulfamique, acide sulfanilique, acide méthanesulfonique et acide p-toluènesulfonique.

5. Composition d'antiperspirant aqueuse selon la revendication 1 ou 2, dans laquelle ledit agent retardateur comprend un groupe -COO⁻ ou -SO₃⁻, et est choisi parmi l'acétate de sodium, citrate de sodium, sorbate de sodium, sulfamate de sodium, alkylbenzènesulfonate de sodium, citrate de potassium, acétate de potassium, sulfamate de potassium, sorbate de potassium, oxalate de potassium, alkylbenzènesulfonate de potassium, acétate d'ammonium, citrate d'ammonium, sulfamate d'ammonium, oxalate d'ammonium, alkylbenzènesulfonate d'ammonium.

6. Composition d'antiperspirant aqueuse selon l'une quelconque des revendications 1 à 5, dans laquelle ladite composition comprend de 0,1 à 40 % en masse dudit sel de phosphate soluble dans l'eau.

7. Composition d'antiperspirant aqueuse selon l'une quelconque des revendications 1 à 6, dans laquelle ladite composition comprend de 0,1 à 40 % en masse dudit sel de non-phosphate soluble dans l'eau.

8. Composition d'antiperspirant aqueuse selon l'une quelconque des revendications 1 à 7, dans laquelle ladite composition comprend de 0,1 à 40 % en masse dudit agent retardateur.

9. Composition d'antiperspirant aqueuse selon l'une quelconque des revendications 1 à 8, dans laquelle ladite composition comprend un actif de déodorant antimicrobien.

10. Composition d'antiperspirant aqueuse selon l'une quelconque des revendications 1 à 9, dans laquelle ladite composition est dans la forme d'une lotion, une crème, un spray, un solide ferme, un solide mou ou une émulsion emballée dans un applicateur à bille.

11. Procédé de réduction de la transpiration comprenant une étape d'application topique d'une composition selon l'une quelconque des revendications 1 à 10.

12. Procédé selon la revendication 11, dans laquelle ladite composition est appliquée sous les bras.

13. Procédé selon la revendication 11 ou 12, dans laquelle ledit sel de phosphate insoluble dans l'eau bloque les canaux sudoripares partiellement ou complètement de manière non-permanente.

14. Utilisation d'une composition selon l'une quelconque des revendications 1 à 10 pour la réduction de la transpiration corporelle.

15. Utilisation selon la revendication 14, dans laquelle lors de ladite utilisation les canaux sudoripares présents dans la peau deviennent partiellement ou complètement bloqués de manière non-permanente.
